# FASCICULE DE BREVET EUROPEEN

(11) **EP 2 054 009 B1**
(45) Date de publication et mention de la délivrance du brevet: **26.09.2012**
(21) Numéro de dépôt: 07823374.9
(22) Date de dépôt: 31.07.2007
(51) Int. Cl.: A61J 15/00

(54) **SONDE DE GASTROSTOMIE PERCUTANEE A COLLERETTE INTERNE UNIQUE BIODEGRADABLE**
PERKUTANE GASTROSTOMIESONDE MIT AUFBLASBAREM BALLON UND BIOLOGISCH ABBAUBAREM VERANKERUNGSMITTEL
PERCUTANEOUS GASTROSTOMY PROBE INCLUDING A SINGLE BIODEGRADABLE INTERNAL FLANGE

(30) Priorité: 02.08.2006 FR 0607074
(43) Date de publication de la demande: 06.05.2009
(73) Titulaire: Medwin France, 34240 Lamalou les Bains (FR)
(72) Inventeur: RENAUX, Serge, F-34240 Lamalou les Bains (FR)
(74) Mandataire: Gosse, Michel
(86) Numéro de dépôt international: PCT/FR2007/001320
(87) Numéro de publication internationale: WO 2008/015337

(56) Documents cités:
- WO-A-99/17708
- WO-A-03/090633
- GB-A- 2 141 346
- US-A1- 2004 097 986

## Description

### DOMAINE DE L'INVENTION

La présente invention se rapporte aux sondes de gastrostomie percutanée endoscopique (GPE) et radiologique (GPR) permettant l'accès direct à la cavité gastrique pour une alimentation entérale. Une telle sonde est connue de WO99/17708.

### ARRIERE PLAN TECHNOLOGIQUE

La gastrostomie percutanée est actuellement la voie de référence pour l'alimentation entérale prolongée. La simplicité et la rapidité de la technique, l'évolution du matériel font que les gastro-entérologues ou les radiologues sont de plus en plus sollicités et que la pose est accessible à tout endoscopiste ou radiologue.

Il existe deux techniques de pose :
- la technique « Pull » par voie endoscopique, principalement utilisée par les gastro-entérologues : les kits de GPE stériles comprennent généralement un trocart de ponction, un fil métallique double brin, une sonde de gastrostomie tubulaire, un moyen de rétention interne, du type collerette, une collerette de fixation externe : idéalement, une pince à corps étrangers type dent de rat ou crocodile est utilisée pour saisir le fil intragastrique, une anse diathermique ou une pince à biopsie peuvent également être utilisées ;
- la technique « Push » par la paroi abdominale, principalement utilisée par les radiologues : dans ce cas des ancres permettent d'arrimer l'estomac à la paroi abdominale le temps nécessaire à la formation d'adhérences entre la partie externe de l'estomac et la paroi abdominale, une sonde à ballonnet est alors positionnée à travers la paroi abdominale au moyen d'un dilatateur et d'une canule pelable.

Les sondes sont généralement en silicone ou polyuréthane, matériaux inertes et bien tolérés. Différents calibres ou charrières sont disponibles, les sondes de petit calibre s'obstruant plus facilement.

Il existe des sondes extractibles et non extractibles.

Les sondes non extractibles, pour être remplacées, doivent être sectionnées au ras de l'orifice cutané. Le dispositif interne est ensuite poussé dans l'estomac.

Le moyen de rétention interne peut être soit récupéré par endoscopie, opération qui peut s'avérer délicate, soit évacué par les voies naturelles avec des risques d'occlusion et de perforation intestinale.

L'avantage des sondes non extractibles réside dans leur collerette interne relativement rigide résistant donc bien à une tentative d'arrachement par un patient agité.

Les sondes extractibles ont une collerette interne rétractable, ou un système de rétention dégonflable, permettant leur ablation par l'orifice cutané par traction ferme. L'avantage de ces systèmes souples est de pouvoir franchir une sténose haute, d'éviter une endoscopie mais elles ont moins de résistance à l'arrachement.

Le choix entre sondes extractibles et non extractibles en silicone ou polyuréthane dépend de l'indication, en tenant compte des avantages et inconvénients de chaque type de sonde.

Les sondes extractibles en silicone conviennent pour une nutrition entérale temporaire. Les sondes non extractibles ou en polyuréthane sont plus adaptées pour une nutrition entérale définitive ou chez un patient agité.

Le remplacement de la sonde de gastrostomie peut s'avérer nécessaire en cas d'obstruction, de détérioration du tube (fissuration, porosité, dilatation, colonisation par des candida).

La majorité des dispositifs de remplacement sont des sondes à ballonnet gonflable à l'eau, en silicone. Leur bonne adéquation avec le milieu gastrique et une collerette de rétention externe permet une bonne sécurité d'emploi.

Il existe également le bouton de gastrostomie plus court et à même la peau qui, du fait de son avantage esthétique et de son confort, est indiqué chez le sujet jeune ou ambulatoire. Il ne peut cependant que difficilement être mis en première intention et vient le plus souvent en remplacement d'une sonde déjà positionnée dans l'estomac.

La sonde selon l'invention est du type comprenant :
a) une tubulure destinée à traverser les parois stomacale et abdominale du sujet ;
b) un moyen de rétention interne, du type collerette destiné à être solidarisé à ladite tubulure et à être maintenu plaqué contre la face interne de la paroi stomacale ;
c) une collerette externe, traversée par ladite tubulure, destinée à être plaquée contre la face externe de la paroi abdominale et à exercer, en coopération avec le moyen de rétention interne, une pression apte à plaquer la paroi stomacale contre la paroi abdominale dans la zone de la stomie.

### RESUME DE L'INVENTION

L'invention vise à réaliser une sonde d'après la revendication 1 et destinée à mettre en oeuvre une solution inédite et originale apte à éliminer les inconvénients susmentionnés.

Elle concerne à cet effet, une sonde de gastrostomie percutanée endoscopique qui se caractérise essentiellement en ce que le moyen de rétention interne est constitué d'une collerette unique entièrement réalisée en un copolymère biodégradable dont la nature des polymères utilisés, leur dosage et leur masse molaire en nombre ainsi que l'épaisseur de ladite collerette, sont déterminés pour obtenir :
- d'une part, un moyen de rétention qui combine des propriétés de souplesse facilitant sa mise en place par voie endoscopique et élastomères lui permettant de reprendre sa forme initiale lorsqu'il est plaqué contre la paroi stomacale ;
- d'autre part, une liaison collerette et tubulure de la sonde qui possède un seuil de rupture à l'arrachement fonction du temps de formation de la stomie.

Le copolymère utilisé est choisi avantageusement parmi les triblocs PLA - PEG - PLA et PLA GA- PEG -PLA GA.

La biodégradation des collerettes internes constitue l'un des avantages essentiels de l'invention en supprimant :
- les risques d'occlusion aux conséquences mortelles ;
- l'utilisation d'un endoscope qui nécessite une anesthésie.

La période de cicatrisation optimale de la stomie est d'environ 21 jours. Un délai trop court conduirait à une mauvaise cicatrisation de celle-ci avec toutes les conséquences qui pourraient en résulter principalement lors de l'introduction d'une sonde de remplacement.

Les incidents qui en résultent concernent essentiellement l'incarcération de la collerette entre les parois, ou le passage accidentel, dans la cavité abdominale, des produits nutritifs administrés. Ces incidents semblent liés à l'ablation en force de la sonde de gastrostomie au travers de la stomie, conduisant à la désolidarisation de la collerette et provoquant la dilacération de la stomie. Le positionnement de la sonde de remplacement est dans ce cas aléatoire.

### PRESENTATION DES FIGURES

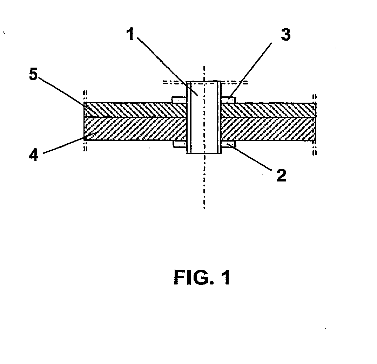

Les caractéristiques et les avantages de l'invention vont apparaître plus clairement à la lecture de la description détaillée qui suit d'au moins un mode de réalisation préféré de celle-ci donné à titre d'exemple non limitatif et représenté au dessin annexé (figure unique) qui représente, en coupe, une vue partielle d'une sonde pourvue d'une collerette interne unique prenant en « sandwich » les parois stomacale et abdominale.

### DESCRIPTION DETAILLEE DE L'INVENTION

La sonde de gastrostomie percutanée représentée est du genre comprenant :
- une tubulure (1) destinée à traverser les parois stomacale (4) et abdominale (5) du sujet ;
- un moyen de rétention interne (2) destiné à être solidarisé à la tubulure (1) et à être maintenu plaqué contre la face interne de ladite paroi stomacale (4) ;
- une collerette externe (3), traversée par la tubulure (1), destinée à être plaquée contre la face externe de ladite paroi abdominale (5) et à exercer, en coopération avec le moyen de rétention interne (2), une pression apte à plaquer la paroi stomacale (4) contre la paroi abdominale (5) dans la zone de la stomie.

Le moyen de rétention interne est constitué d'une collerette unique (2) entièrement réalisée en un copolymère biodégradable dont la nature des polymères utilisés, leur dosage et leur masse molaire en nombre ainsi que l'épaisseur de ladite collerette, sont déterminés pour obtenir :
- d'une part, un moyen de rétention qui combine des propriétés de souplesse facilitant sa mise en place par voie endoscopique et élastomères lui permettant de reprendre sa forme initiale lorsqu'il est plaqué contre la paroi stomacale ;
- d'autre part, une liaison collerette (2) et tubulure (1) qui possède un seuil de rupture à l'arrachement fonction du temps de formation de la stomie.

La vitesse de biodégradabilité du moyen de rétention est programmée de sorte que les caractéristiques mécaniques de celui-ci soient maintenues au moins jusqu'à l'adhésion des parois stomacale et abdominale entre elles.

La collerette (2), qui est pourvue d'une ouverture pour le passage de la tubulure (1), peut être solidarisée à cette dernière par collage, soudage, sertissage ou par tout autre moyen connu.

La tubulure (1) est généralement réalisée en un matériau biocompatible non biodégradable comme par exemple en silicone ou en polyuréthane.

La liaison de la collerette de rétention interne (2) à la tubulure (1) est conçue pour se désolidariser de cette dernière sous l'effet d'une traction externe bien définie sur celle-ci.

Le choix du polymère ou copolymère biodégradable adapté à l'application médicale considérée a fait l'objet de tests consistant à synthétiser divers polymères ou copolymères en des échantillons, immergés dans un modèle de fluide gastrique, ayant des dimensions similaires à celles des moyens de rétention internes biodégradables concernés, capables de se dégrader dans un délai bien déterminé (notamment compris entre 1 et 3 mois), ayant les caractéristiques physiques et mécaniques requises en termes d'élasticité ou de dureté, de changement de forme, de gonflement due à la prise d'eau, de décomposition...

Des tests ont été effectués sur des échantillons réalisés avec les divers polymères ou copolymères des types :
- poly acide lactique comme le PLA 50 (Mn = 21000g/mol) et le PLA 50 (Mn = 46000 g/mol) ;
- poly acide lactique-acide glycolique comme le PLA 37,5 - GA 25 (Mn = 39000 g/mol) ;
- triblocs PLA - PEG (poly éthylène glycol) - PLA comme le PLA 50 - PEG (20000) - PLA 50 (Mn = 277760 g/mol), le PLA 50 - PEG (20000) - PLA 50 (Mn = 100600 g/mol), le PLA 50 - PEG (6000) - PLA 50 (Mn = 56400 g/mol), le PLA 96 - PEG 12000
- PLA 96 (Mn = 68311 g/mol), le PLA 96 - PEG 8000 - PLA 96 (Mn = 71684 g/mol) et les PLA GA - PEG - PLA GA.

C'est dans la catégorie des copolymères PLA 96 - PEG 12000 - PLA 96 (Mn = 68311 g/mol), PLA 96 - PEG 8000 - PLA 96 (Mn = 71684 g/mol) et PLA GA - PEG - PLA GA, que le choix s'est porté pour réaliser la collerette interne qui doit obéir aux critères suivants :
- un dosage plus élevé en PLA (L), supérieur à 60, pour obtenir le meilleur compromis entre les qualités élastomères (mémoire de forme) et de souplesse ;
- un dosage approprié de la masse molaire (Mn) du PEG pour une meilleure gestion du temps de dégradation ;
- des dimensions appropriées de la collerette : épaisseur comprise avantageusement entre 1 et 3 mm car celle-ci joue également sur la vitesse de biodégradabilité.
Des composants ayant pour effet de modifier les caractéristiques mécaniques et la vitesse de dégradation ainsi que la tolérance de l'organisme, peuvent être rajoutés auxdits copolymères

Bien entendu, l'invention n'est pas limitée aux modes de réalisation décrits et représentés pour lesquels l'homme de métier pourra prévoir d'autres variantes, en particulier dans les types de sondes utilisées et les matériaux constitutifs des sous ensembles desdites sondes à la condition que la collerette interne soit entièrement biodégradable.

## Revendications

1. Sonde de gastrostomie percutanée comprenant :
a) une tubulure (1) destinée à traverser les parois stomacale (4) et abdominale (5) du sujet ;
b) un moyen de rétention interne (2) destiné à être solidarisé à ladite tubulure (1) et à être maintenu plaqué contre la face interne de ladite paroi stomacale (4) ;
c) une collerette externe (3), traversée par la tubulure (1), destinée à être plaquée contre la face externe de ladite paroi abdominale (5) et à exercer, en coopération avec le moyen de rétention interne (2), une pression apte à plaquer la paroi stomacale (4) contre la paroi abdominale (5) dans la zone de la stomie ;
**caractérisée en ce que** le moyen de rétention interne est constitué d'une collerette unique (2) entièrement réalisée en un copolymère biodégradable constitué d'un tribloc PLA - PEG - PLA qui possède, d'une part, des propriétés de souplesse facilitant sa mise en place par voie endoscopique et des propriétés élastomères lui permettant de reprendre sa forme initiale lorsqu'il est plaqué contre la paroi stomacale et, d'autre part, une liaison collerette (2) et tubulure (1) présentant un seuil de rupture à l'arrachement fonction du temps de formation de la stomie.

2. Sonde, selon la revendication 1, **caractérisée en ce que** le copolymère utilisé est constitué d'un tribloc PLA GA- PEG - PLA GA.

3. Sonde, selon la revendication 1, **caractérisé en ce que** le copolymère utilisé est constitué du tribloc PLA 96 - PEG 12000 - PLA 96 (Mn = 68311 g/mol).

4. Sonde, selon la revendication 1, **caractérisé en ce que** le copolymère utilisé est constitué du tribloc PLA 96 - PEG 8000 - PLA 96 (Mn = 71684 g/mol).

5. Sonde, selon la revendication 1, **caractérisé en ce que** la collerette (2) possède une épaisseur comprise entre 1 et 3 mm.

## Claims

1. Percutaneous gastrostomy tube, including:
a) a tubing (1) intended to pass through the stomach (4) and abdominal (5) walls of the subject;
b) internal holding means (2) intended to be secured to said tubing (1) and to be kept pressed against the internal face of said stomach wall (4);
c) an external collar (3), through which the tubing (1) passes, intended to be pressed against the external face of said abdominal wall (5) and to exert, in cooperation with the internal holding means (2), a pressure capable of pressing the stomach wall (4) against the abdominal wall (5) in the area of the stoma;
**characterized in that** the internal holding means consist of a single collar (2) made entirely of a biodegradable copolymer consisting of a PLA - PEG - PLA triblock that has, on the one hand, flexibility properties facilitating its endoscopic placement and elastomer properties enabling it to return to its initial shape when it is pressed against the stomach wall, and, on the other hand, a collar (2) and tubing (1) connection having a tearing threshold dependent on the stoma formation time.

2. Probe, according to claim 1, **characterized in that** the copolymer used consists of a PLA GA- PEG - PLA GA triblock.

3. Probe, according to claim 1, **characterized in that** the copolymer used consists of triblock PLA 96 - PEG 12000 - PLA 96 (Mn = 68311 g/mol).

4. Probe, according to claim 1, **characterized in that** the copolymer used consists of triblock PLA 96 - PEG 8000 - PLA 96 (Mn = 71684 g/mol).

5. Probe, according to claim 1, **characterized in that** the collar (2) has a thickness between 1 and 3 mm.

## Patentansprüche

1. Perkutane Gastrostomiesonde, umfassend:
a) einen Schlauch (1), der dazu ausgelegt ist, die Magen (4) und Bauchwand (5) des Patienten zu durchqueren;
b) ein internes Rückhaltemittel (2), das dazu ausgelegt ist, mit dem Schlauch (1) fest verbunden zu sein und gegen die innere Seite der Magenwand (4) gedrückt gehalten zu werden;
c) einen externen Kragen (3), durchquert von dem Schlauch (1), dazu ausgelegt, gegen die äußere Seite der Bauchwand (5) gedrückt zu werden und, in Zusammenarbeit mit dem internen Rückhaltmittel (2), einen Druck auszuüben, der dazu geeignet ist, die Magenwand (4) gegen die Bauchwand (5) im Bereich der Ostomie zu drücken;
**dadurch gekennzeichnet dass** das interne Rückhaltemittel aus einem einzigen Kragen (2) besteht, der vollständig aus einem biologisch abbaubaren Copolymer hergestellt ist, bestehend aus einem Triblock PLA - PEG - PLA, der einerseits Eigenschaften der Geschmeidigkeit aufweist, die seine Anbringung auf endoskopischem Weg ermöglichen, und elastomere Eigenschaften, die es ihm ermöglichen, seine ursprüngliche Form wieder einzunehmen, wenn er gegen die Magenwand gedrückt wird, und andererseits eine Verbindung zwischen Kragen (2) und Schlauch (1), die eine Bruchschwelle aufweist, die eine Funktion der Zeit der Bildung der Ostomie ist.

2. Sonde nach Anspruch 1, **dadurch gekennzeichnet, dass** das verwendete Copolymer aus einem Triblock PLA GA- PEG - PLA GA besteht.

3. Sonde nach Anspruch 1, **dadurch gekennzeichnet, dass** das verwendete Copolymer aus dem Triblock PLA 96 - PEG 12000 - PLA 96 (Mn = 68311 g/mol) besteht.

4. Sonde nach Anspruch 1, **dadurch gekennzeichnet, dass** das verwendete Copolymer aus dem Triblock PLA 96 - PEG 8000 - PLA 96 (Mn = 71684 g/mol) besteht.

5. Sonde nach Anspruch 1, **dadurch gekennzeichnet, dass** der Kragen (2) eine Dicke zwischen 1 und 3 mm aufweist.
